# EUROPÄISCHE PATENTSCHRIFT

(11) **EP 3 441 005 B1**
(45) Veröffentlichungstag und Bekanntmachung des Hinweises auf die Patenterteilung: **06.04.2022**
(21) Anmeldenummer: 17185862.4
(22) Anmeldetag: 11.08.2017
(51) Int. Cl.: A61B 6/03, A61B 6/00

(54) **ANALYSE VON LÄSIONEN MIT HILFE DER MULTI-ENERGIE-CT-BILDGEBUNG**
ANALYSIS OF LESIONS WITH THE AID OF MULTI-ENERGY CT IMAGING
ANALYSE DES LÉSIONS AU MOYEN DE L'IMAGERIE CT MULTI-ÉNERGIE

(43) Veröffentlichungstag der Anmeldung: 13.02.2019
(73) Patentinhaber: Siemens Healthcare GmbH, 91052 Erlangen (DE)
(72) Erfinder: Krauß, Bernhard, 90559 Burgthann (DE); Schmidt, Bernhard, 90766 Fürth (DE)

(56) Entgegenhaltungen:
- WO-A2-2014/141163
- US-A1- 2010 135 557
- US-A1- 2013 287 260
- ANTHONY P.H. BUTLER ET AL: "Processing of spectral X-ray data with principal components analysis", NUCLEAR INSTRUMENTS & METHODS IN PHYSICS RESEARCH. SECTION A: ACCELERATORS, SPECTROMETERS, DETECTORS, AND ASSOCIATED EQUIPMENT, Bd. 633, 1. Mai 2011 (2011-05-01), Seiten S140-S142, XP055452770, NL ISSN: 0168-9002, DOI: 10.1016/j.nima.2010.06.149

## Beschreibung

Die Erfindung betrifft ein Verfahren zur Analyse eines Auswertungsbereichs eines Untersuchungsbereichs eines Untersuchungsobjekts. Zudem betrifft die Erfindung eine Bildauswerteeinrichtung. Überdies betrifft die Erfindung ein Computertomographiesystem.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems (CT-Systems) akquiriert werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden, die die Röntgenschwächung des Patienten in dieser Durchstrahlungsrichtung beschreiben.

Die erzeugten Projektionsmessdaten, kurz auch als Projektionsdaten bezeichnet, sind insbesondere von der Bauart des Röntgendetektors abhängig. Röntgendetektoren weisen gewöhnlich eine Mehrzahl an Detektionseinheiten auf, die meist in Form eines regelmäßigen Pixelarrays angeordnet sind. Die Detektionseinheiten erzeugen jeweils für auf die Detektionseinheiten auftreffende Röntgenstrahlung ein Detektionssignal, welches zu bestimmten Zeitpunkten hinsichtlich Intensität und spektraler Verteilung der Röntgenstrahlung analysiert wird, um Rückschlüsse auf das Untersuchungsobjekt zu erhalten und Projektionsmessdaten zu erzeugen.

Bei einigen Arten von CT-Bildgebungsverfahren werden mehrere Bildaufnahmen mit Röntgenstrahlung mit unterschiedlichen Röntgenenergiespektren von ein und demselben Untersuchungsbereich eines Patienten durchgeführt. Diese Vorgehensweise wird auch als Multi-Energie-CT-Aufnahme bezeichnet; der Spezialfall mit zwei Röntgenspektren ist als Zwei-Spektren-CT oder Dual-Energy-CT bekannt. Eine solche Multi-Energie-CT-Aufnahme kann zum Beispiel mit Hilfe von mehreren CT-Bildaufnahmen nacheinander mit unterschiedlicher Röhrenspannung erfolgen. Man kann auch simultan Aufnahmen mit unterschiedlichen Energiespektren realisieren, wenn man einen energieempfindlichen Detektor verwendet und bei einer einzigen CT-Bildaufnahme gleichzeitig Röntgenschwächungsdaten mit unterschiedlichen effektiven Spektren aufnimmt. Diese Vorgehensweise kann zum Beispiel mit Hilfe von quantenzählenden Detektoren oder mehrlagigen Detektoren realisiert werden. Alternativ dazu kann man auch zwei Röntgenröhren und zwei gewöhnliche Detektoren simultan innerhalb der gleichen Gantry betreiben. Ein solcher Aufbau wird als Dual-Source-CT bezeichnet und kann für Dual Energy-CT-Aufnahmen verwendet werden, indem man die beiden Röntgenröhren mit unterschiedlicher Spannung und/oder Filterung betreibt.

CT-Bildgebungsverfahren werden auch zur Analyse von Läsionen und damit insbesondere zur Detektion von Tumoren eingesetzt. Die Analyse erfolgt auf Basis der Ermittlung von Abschwächungswerten der Bilddaten im Bereich der Läsionen. Allerdings treten insbesondere bei klein dimensionierten Läsionen bei der Auswertung der Bilddaten sogenannte Partialvolumeneffekte auf, die es erschweren, einer Läsion einen korrekten Abschwächungswert zuzuordnen, insbesondere dann, wenn das die Läsion umgebende Material einen deutlich höheren Abschwächungswert aufweist. Dies ist zum Beispiel bei der Charakterisierung hypodenser Läsionen in der Leber oder der Niere der Fall. Hypodense Läsionen sind Gewebeveränderungen mit einer verminderten Dichte im Vergleich zur Umgebung.

Die Veröffentlichung von Anthony P.H. Butler et al. mit dem Titel "Processing of spectral X-ray data with principal components analysis" (in NUCLEAR INSTRUMENTS & METHODS IN PHYSICS RESEARCH; Bd. 633; Seiten S140-S142), offenbart eine Material-Dekomposition einer zweidimensionalen Verteilung spektraler Abschwächungswerte mittels Hauptkomponentenanalyse.

Herkömmlich müssen daher in solchen Fällen oft weitere Bildgebungsmodalitäten, wie zum Beispiel die MR-Bildgebung für eine Zusatzuntersuchung herangezogen werden. Allerdings bedeutet dies einen erhöhten Aufwand und eine zusätzliche Belastung für den Patienten.

Es besteht also das Problem, eine vereinfachte Vorgehensweise für die Auswertung von relativ kleinen, schwer von der Umgebung abzugrenzenden Untersuchungsbereichen zu entwickeln. Diese Aufgabe wird durch ein Verfahren zur Analyse eines Auswertungsbereichs eines Untersuchungsbereichs gemäß Patentanspruch 1, eine Bildauswerteeinrichtung gemäß Patentanspruch 13 und ein Computertomographiesystem gemäß Patentanspruch 14 gelöst.

Bei dem erfindungsgemäßen Verfahren zur Analyse eines Auswertungsbereichs in einem Untersuchungsbereich eines Untersuchungsobjekts werden zwei Projektionsmessdatensätze erfasst. Der im Untersuchungsbereich enthaltene Auswertungsbereich sollte bei Anwesenheit einer Läsion so gewählt werden, dass die Läsion und der Umgebungsbereich innerhalb des Auswertungsbereichs ein etwa gleich großes Volumen umfassen.

Die Projektionsmessdatensätze werden mit Hilfe einer Multi-Energie-CT-Aufnahme mit unterschiedlichen effektiven Röntgenenergiespektren von dem Untersuchungsbereich des Untersuchungsobjekts erzeugt.

Weiterhin werden zwei Bilddatensätze auf Basis der zwei Projektionsmessdatensätze rekonstruiert. Zudem werden Abschwächungswertepaare, welche jeweils einem gemeinsamen Bildpunkt der beiden Bilddatensätze in dem Auswertungsbereich zugeordnet sind, ermittelt. Die Abschwächungswertepaare werden durch eine zweidimensionale Verteilung der Abschwächungswertepaare repräsentiert. In Bezug auf die spektrale Information unterscheidet sich eine hypodense Läsion in der Regel von den Materialien des Umgebungsbereichs, denn üblicher Weise weist das umgebende Material einen hohen CT-Wert auf, weil dort mehr Atome mit hoher Ordnungszahl anwesend sind. Dagegen befinden sich in dem Bereich der Läsion nur wenige Atome mit einer hohen Ordnungszahl, so dass dieser Bereich einen niedrigeren CT-Wert aufweist. Mit Hilfe der spektralen Bildgebung lassen sich nun Bilder nach unterschiedlichen Atomarten getrennt aufnehmen und auswerten. Die entsprechenden herkömmlichen Verfahren zur Materialzerlegung sind allgemein bekannt, können aber das Problem von Partialvolumeneffekten nicht lösen: Diese sind eine Folge der begrenzten räumlichen Auflösung des CT-Bildes und selbst in reinen Materialbildern kann deshalb die Läsion fälschlich eine Anreicherung mit Kontrastmittel zeigen, wenn das umgebende Material viel Kontrastmittel enthält.

Um dieses Problem zu umgehen, wird erfindungsgemäß stattdessen direkt im Raum der Abschwächungswerte eine die zweidimensionale Verteilung der Abschwächungswertepaare charakterisierende Gerade ermittelt. Typischerweise sind die Abschwächungswertepaare einer Läsion einschließlich ihrer Oberfläche annähernd in Form einer Ellipse zweidimensional verteilt. Im Grenzfall können die Abschwächungswerte für zwei unterschiedliche Materialien zwei voneinander getrennte Kreise bilden, wobei einige Voxel aus dem Übergangsbereich zwischen Läsion und Umgebung zwischen diesen Kreisen liegen.

Die Verteilung in Form einer Ellipse wird auch dadurch verursacht, dass bei der Bildaufnahme des Untersuchungsbereichs auf Grund von Partialvolumeneffekten die CT-Werte der Läsion an der Oberfläche mit den CT-Werten des umgebenden Materials linear gemischt werden. Dabei kann man davon ausgehen, dass die Bildschärfe in beiden Bildern annähernd gleich ist. Unter der Annahme, dass genau zwei Materialien im Kontakt miteinander stehen, bilden die Abschwächungswerte der Bildpunkte dann eine Gerade. Allerdings kann insbesondere bei klinischen Untersuchungen in der Regel keine hohe Strahlendosis angewendet werden, so dass in beiden Bildern in der Regel erhebliches Bildrauschen vorliegt. Daher tritt eine flächige Verteilung annähernd in Form einer Ellipse auf. Die Gerade verläuft dann ungefähr in Richtung der großen Halbachse dieser Ellipse. Der Verlauf der Geraden kann zum Beispiel mit Hilfe einer Ausgleichsrechnung, beispielsweise als Regressionsgerade, oder durch Hauptkomponentenanalyse ermittelt werden. Der Verlauf der Geraden repräsentiert die Absorptionseigenschaften der Läsion ebenso wie die Absorptionseigenschaften des umgebenden Materials. Schließlich wird eine den Auswertungsbereich betreffende Materialeigenschaft auf Basis der ermittelten Geraden und einer die gesuchte Materialeigenschaft betreffenden Nebenbedingung ermittelt. Die Nebenbedingung enthält bekannte Informationen bzw. Forderungen, welche von der Materialeigenschaft erfüllt werden müssen. Beispielsweise können das bestimmte Arten von Materialien sein, welche für einen Untersuchungsbereich in Frage kommen. Die Materialeigenschaft selbst kann zum Beispiel eine Materialart bzw. eine Verteilung eines bestimmten Materials sein.

Die erfindungsgemäße Bildauswerteeinrichtung weist eine Projektionsmessdatenerfassungseinheit zum Erfassen von mindestens zwei Projektionsmessdatensätzen auf. Die Projektionsmessdatensätze werden mit Hilfe einer Multi-Energie-CT-Aufnahme mit unterschiedlichen effektiven Röntgenenergiespektren von dem Untersuchungsbereich des Untersuchungsobjekts aufgenommen. Teil der erfindungsgemäßen Bildauswerteeinrichtung ist eine Bilddatenrekonstruktionseinheit, welche dazu eingerichtet ist, mindestens zwei Bilddatensätze auf Basis der mindestens zwei Projektionsmessdatensätze zu rekonstruieren.

Falls mehr als zwei Röntgenspektren verwendet werden, sind Methoden bekannt, um diese ohne Verlust von Informationen auf genau zwei Rohdaten- bzw. Bilddatensätze zu reduzieren und anschließend das erfindungsgemäße Analyse- bzw. Auswertungsverfahren anzuwenden. Ein Spezialfall ist auch, dass als Teil der Bildrekonstruktion eine rohdatenbasierte Methode verwendet werden kann, um zum Beispiel sogenannte monoenergetische Bilder oder Basismaterialbilder zu erzeugen. Das erfindungsgemäße Verfahren kann auch diese Bilder verwenden um aus diesen Bildern Abschwächungswertepaare zu entnehmen. Ein Verfahren zur ʺReduktion der Anzahl spektraler Kanäle in der Multi-Energie-CT-Bildgebung" ist in der deutschen Patentanmeldung mit dem amtlichen Aktenzeichen DE 10 2017 200 032.5 beschrieben.

Die erfindungsgemäße Bildauswerteeinrichtung umfasst außerdem eine Paar-Ermittlungseinheit, welche dazu eingerichtet ist, Abschwächungswertepaare, welche jeweils einem gemeinsamen Bildpunkt der beiden Bilddatensätze in dem Auswertungsbereich des Untersuchungsbereichs zugeordnet sind, anhand der beiden unterschiedlichen Bilddatensätze zu ermitteln. Hierzu können zum Beispiel die beiden Bilddatensätze mit unterschiedlichen Röntgenenergiespektren aufeinander registriert werden bzw. jeweils an denselben Bildkoordinaten positionierte Bildpunkte der beiden unterschiedlichen Bilddatensätze können einander zugeordnet werden. Weiterhin ist Teil der erfindungsgemäßen Bildauswerteeinrichtung eine Geraden-Ermittlungseinheit. Die Geraden-Ermittlungseinheit ist dazu eingerichtet, eine eine zweidimensionale Verteilung der Abschwächungswerte charakterisierende Gerade zu ermitteln. Wie bereits erwähnt, kann der Verlauf der Geraden zum Beispiel mit Hilfe einer Ausgleichsrechnung, beispielsweise als Regressionsgerade, oder durch Hauptkomponentenanalyse ermittelt werden. Die erfindungsgemäße Bildauswerteeinrichtung umfasst überdies eine Materialeigenschaft-Ermittlungseinheit zum Ermitteln einer den Auswertungsbereich des Untersuchungsbereichs betreffenden Materialeigenschaft auf Basis der ermittelten Geraden und einer die gesuchte Materialeigenschaft betreffenden Nebenbedingung. Die erfindungsgemäße Bildauswerteeinrichtung teilt die Vorteile des erfindungsgemäßen Verfahrens zur Analyse eines Auswertungsbereichs eines Untersuchungsbereichs eines Untersuchungsobjekts.

Das erfindungsgemäße Computertomographiesystem, ein Multi-Energie-CT-System, weist eine Scaneinheit zum Abtasten eines Untersuchungsbereichs eines zu untersuchenden Objekts, eine Steuerungseinrichtung zum Ansteuern der Scaneinheit und eine erfindungsgemäße Bildauswerteeinrichtung auf. In diesem Fall werden die von dem Computertomographiesystem aufgenommenen Multi-Energie-Projektionsmessdaten von einem Untersuchungsbereich eines Untersuchungsobjekts von der Bildauswerteeinrichtung dahingehend verarbeitet, dass daraus auf Basis von rekonstruierten Bilddatensätzen mit unterschiedlichem Röntgenenergiespektrum Materialeigenschaften des Auswertungsbereichs des Untersuchungsbereichs analysiert werden. Da die Bildauswerteeinrichtung direkt in das Computertomographiesystem integriert ist, werden keine zusätzlichen Geräte außerhalb des CT-Systems benötigt, um eine Ermittlung der Materialeigenschaften durchzuführen.

Die wesentlichen Komponenten der erfindungsgemäßen Bildauswerteeinrichtung können zum überwiegenden Teil in Form von Softwarekomponenten ausgebildet sein. Dies betrifft insbesondere Teile der Projektionsmessdatenerfassungseinheit, der Bilddatenrekonstruktionseinheit, der Paar-Ermittlungseinheit, der Geraden-Ermittlungseinheit und der Materialeigenschaft-Ermittlungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Steuerungseinrichtungen von Computertomographiesystemen oder auch andere zur Analyse und Auswertung genutzte Rechnersysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines erfindungsgemäßen Computertomographiesystems oder einer anderen Rechnereinheit ladbar ist, mit Programmabschnitten, um alle Schritte des erfindungsgemäßen Verfahrens auszuführen, wenn das Computerprogramm in dem Computertomographiesystem oder einer anderen zur Auswertung der von dem Computertomographiesystem erzeugten Projektionsmessdaten und Bilddaten verwendeten Rechnereinheit ausgeführt wird.

Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen.

Zum Transport zur Speichereinrichtung des Computertomographiesystems oder der genannten Rechnereinheit und/oder zur Speicherung an dem Computertomographiesystem oder der genannten Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit der einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zur Analyse eines Auswertungsbereichs eines Untersuchungsbereichs eines Untersuchungsobjekts umfasst der Auswertungsbereich eine Läsion, vorzugsweise eine hypodense Läsion, und zusätzlich die Läsion umgebendes Gewebe. Die für das Verfahren relevanten Läsionen haben häufig kleinere Abmessungen als etwa 1 cm. Typische Schichtdicken von CT-Bildddaten liegen häufig bei 3 bis 5 mm. Daher lassen sich oft Umgebungsbereiche nicht eindeutig von einer Läsion trennen, so dass Information aus den Umgebungsbereichen die Bildinformationen, welche den eigentlichen Läsionen zugeordnet sind, überlagert. Vorteilhaft kann nun mit Hilfe des erfindungsgemäßen Verfahrens die den Läsionen zugeordnete Bildinformation bzw. darauf basierende Informationen über Materialeigenschaften der Läsionen extrahiert werden. Vorteilhaft können also mit dem erfindungsgemäßen Verfahren auch kleine Läsionen zuverlässig analysiert werden. Mit dem erfindungsgemäßen Verfahren können auch Teilbereiche einer Läsion ausgewertet werden.

In einer bevorzugten Ausgestaltung des erfindungsgemäßen Verfahrens zur Analyse eines Auswertungsbereichs eines Untersuchungsbereichs eines Untersuchungsobjekts werden als Nebenbedingung Abschwächungswertepaare unterschiedlicher Kandidaten-Materialien verwendet. Dabei umfasst das Ermitteln einer den Auswertungsbereich betreffenden Materialeigenschaft das Ermitteln eines der Kandidaten-Materialien als Bestandteil des Auswertungsbereichs des Untersuchungsbereichs, dessen Abschwächungswertepaar der Geraden am nächsten kommt. Darunter ist zu verstehen, dass der Abstand des das Abschwächungswertepaar repräsentierenden Punkts in einem Schaubild zu der genannten Geraden im Vergleich zu anderen Kandidaten-Materialien minimal ist.

Es werden also bekannte Absorptionseigenschaften von Materialien die in dem Untersuchungsbereich vermutet werden, mit der Geraden, welche die Verteilung der ermittelten Abschwächungswertepaare repräsentiert, verglichen und das Material, dessen Absorptionseigenschaften dem Absorptionsverhalten der ermittelten Geraden am nächsten kommt, als in dem Auswertungsbereich vorhandenes Material detektiert.

Bevorzugt wird bei dem erfindungsgemäßen Verfahren zur Analyse eines Auswertungsbereichs eines Untersuchungsbereichs eines Untersuchungsobjekts als Nebenbedingung eine Gerade verwendet, welche Abschwächungswertepaare unterschiedlicher Arten von Kandidaten-Gewebestrukturen repräsentiert. Das Ermitteln einer den Auswertungsbereich des Untersuchungsbereichs betreffenden Materialeigenschaft umfasst dann das Ermitteln eines realen Abschwächungswertepaares. Das reale Abschwächungswertepaar wird als Schnittpunkt der die Verteilung der Abschwächungswerte charakterisierenden Geraden und der Geraden, welche Abschwächungswertepaare unterschiedlicher Arten von Kandidaten-Gewebestrukturen repräsentiert, ermittelt.

Im Fall einer Kontrastmittelaufnahme in einer Läsion ist die erhaltene Charakterisierung unter Umständen nicht eindeutig. Zum Beispiel kann ein Lebertumor mit relativ hoher Iod-Aufnahme einen ähnlichen wahrscheinlichsten Schwächungswert aufweisen wie eine gewöhnliche Zyste. In diesem Fall ist es aber möglich, stattdessen die Kontrastmittelaufnahme in der Läsion zu ermitteln. Dabei wird zur Auswertung von einem Auswertungsbereich eines Untersuchungsbereichs eines Untersuchungsobjekts als Nebenbedingung eine Gerade verwendet, welche Abschwächungswertepaare unterschiedlicher Arten von Kandidaten-Gewebsstrukturen repräsentiert.

Dazu werden hochaufgelöste Nativbilddaten von dem Untersuchungsbereich zum Beispiel aus einer Vorgängeruntersuchung benötigt. Die Nativbilddaten sind Bilddaten, welche ohne Kontrastmittel erfasst wurden, so dass zum Beispiel ein Abschwächungswert einer Läsion ohne Kontrastmittel bekannt ist. Falls die Nativbilddaten mit nur einem Spektrum aufgenommen wurden, kann man zum Beispiel zusätzlich annehmen, dass der CT-Wert der Läsion in Abwesenheit von Kontrastmittel unabhängig vom Spektrum ist. Die Nativbilder werden vorzugsweise mit höherer räumlicher Auflösung als die Multi-Energie-Bilddaten aufgenommen.

Auf Basis des Nativbildes wird ein Abschwächungswertepaar auf der Geraden, welche Abschwächungswertepaare unterschiedlicher Arten von Kandidaten-Gewebestrukturen repräsentiert, ermittelt. Das Abschwächungswertepaar charakterisiert dabei den Auswertungsbereich des Untersuchungsbereichs in dem Nativbild. Weiterhin wird eine die Kontrastmittelaufnahme des Untersuchungsbereichs charakterisierende Gerade ermittelt. Diese Gerade verläuft durch den Punkt des den Auswertungsbereich des Untersuchungsbereichs in dem Nativbild charakterisierenden Abschwächungswertepaars. Die Steigung dieser Geraden ergibt sich aus dem Absorptionsverhalten des zur multispektralen Bildgebung eingesetzten Kontrastmittels. Das Ermitteln einer den Auswertungsbereich betreffenden Materialeigenschaft umfasst bei dieser Variante das Ermitteln eines realen Abschwächungswertepaares, welches als Schnittpunkt der die Kontrastmittelaufnahme des Auswertungsbereichs des Untersuchungsbereichs charakterisierenden Geraden und der die Verteilung der Abschwächungswerte charakterisierenden Geraden ermittelt wird. Diese Variante des erfindungsgemäßen Verfahrens kann insbesondere für unterschiedliche Typen von Läsionen in der Leber und der Niere verwendet werden.

In einer besonders vorteilhaften Ausgestaltung des erfindungsgemäßen Verfahrens zur Analyse eines Auswertungsbereichs eines Untersuchungsbereichs eines Untersuchungsobjekts wird auf Basis der ermittelten Materialeigenschaft ermittelt, ob in dem Auswertungsbereich ein Tumor oder eine Zyste vorliegt. Vorteilhaft können unterschiedliche Materialeigenschaften in Tumoren und Zysten zur Differenzierung zwischen diesen Gewebearten genutzt werden.

In einer anderen Variante des erfindungsgemäßen Verfahrens zur Analyse eines Auswertungsbereichs eines Untersuchungsbereichs eines Untersuchungsobjekts wird in Abhängigkeit davon, ob die in der Läsion ermittelte Kontrastmittelaufnahme einen vorbestimmten Schwellwert überschreitet, ermittelt, dass in dem Untersuchungsbereich bzw. dem Auswertungsbereich ein Tumor vorliegt. Vorteilhaft wird bei dieser Variante die Tatsache genutzt, dass Tumore aufgrund einer starken Kontrastmittelaufnahme höhere Abschwächungswerte aufweisen als gutartige Läsionen.

In einer speziellen Ausgestaltung des erfindungsgemäßen Verfahrens zur Analyse eines Auswertungsbereichs eines Untersuchungsbereichs eines Untersuchungsobjekts wird in Abhängigkeit davon, ob der Punkt des realen Abschwächungswertepaares und der Punkt des den Auswertungsbereich des Untersuchungsbereichs in dem Nativbild charakterisierenden Abschwächungswertepaars voneinander entfernt liegen, ermittelt, ob ein Tumor oder eine Zyste in dem Auswertungsbereich vorliegen. Bei dieser Variante kann ermittelt werden, ob die Läsion Kontrastmittel aufnimmt oder nicht. Nimmt sie Kontrastmittel auf, so deutet das darauf hin, dass es ein Tumor ist. Andernfalls kann angenommen werden, dass es sich um eine gutartige Läsion handelt.

In einer weiteren Variante des erfindungsgemäßen Verfahrens zur Analyse eines Auswertungsbereichs eines Untersuchungsbereichs eines Untersuchungsobjekts wird in Abhängigkeit von dem Abstand zwischen dem Punkt des realen Abschwächungswertepaares und dem Punkt des den Auswertungsbereich des Untersuchungsbereichs in dem Nativbild charakterisierenden Abschwächungswertepaars die Art eines vorliegenden Tumortyps ermittelt. Aus dem Absorptionsverhalten von Kontrastmittel des Tumors kann auf diese Art vorteilhaft auf die Art des Tumors geschlossen werden.

Der bei dem erfindungsgemäßen Verfahren abgebildete Auswertungsbereich kann zum Beispiel hinsichtlich einer der folgenden Strukturen untersucht werden:
- eine gewöhnliche Zyste,
- eine eingeblutete Zyste,
- Metastasen,
- Primärtumore.

Mit dem beschriebenen erfindungsgemäßen Verfahren können nun die unterschiedlichen Arten von Tumoren bzw. Gewebeanomalien voneinander unterschieden werden. Beispielsweise weist eine gewöhnliche Zyste Abschwächungswerte von etwa 0 HU (HU = Hounsfield Unit) auf. Dagegen weist eine eingeblutete Zyste in den beiden Bildern, welche bei einer Dual-Energie-CT-Aufnahme erzeugt wurden, annähernd gleiche Abschwächungswerte von 0 und 80 HU auf. Charakteristische Abschwächungswerte für Metastasen und Primärtumore können zum Beispiel klinisch mit Hilfe der Multi-Energie-CT-Bildgebung ermittelt werden. Sobald diese bekannt sind, lassen sich mit den beschriebenen Verfahren Läsionen den unterschiedlichen Typen von Tumoren zuordnen.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zur Analyse eines Auswertungsbereichs eines Untersuchungsbereichs eines Untersuchungsobjekts gemäß einem ersten Ausführungsbeispiel der Erfindung veranschaulicht,
FIG 2 ein Schaubild, welches die Anwendung des in FIG 1 veranschaulichten Verfahrens auf eine Verteilung von Abschwächungswertepaaren von zwei unterschiedlichen spektralen Bilddatensätzen veranschaulicht,
FIG 3 ein Flussdiagramm, welches ein Verfahren zur Analyse eines Auswertungsbereichs eines Untersuchungsbereichs eines Untersuchungsobjekts gemäß einem zweiten Ausführungsbeispiel der Erfindung veranschaulicht,
FIG 4 ein Schaubild, welches die Anwendung des in FIG 3 veranschaulichten Verfahrens auf eine Verteilung von Abschwächungswertepaaren von zwei unterschiedlichen spektralen Bilddatensätzen veranschaulicht,
FIG 5 ein Flussdiagramm, welches ein Verfahren zur Analyse eines Auswertungsbereichs eines Untersuchungsbereichs eines Untersuchungsobjekts gemäß einem dritten Ausführungsbeispiel der Erfindung veranschaulicht,
FIG 6 ein Schaubild, welches die Anwendung des in FIG 5 veranschaulichten Verfahrens auf eine Verteilung von Abschwächungswertepaaren von zwei unterschiedlichen spektralen Bilddatensätzen veranschaulicht,
FIG 7 ein Blockdiagramm, mit dem eine Bildauswerteeinrichtung gemäß einem Ausführungsbeispiel der Erfindung veranschaulicht wird,
FIG 8 ein Computertomographiesystem mit einer Bildauswerteeinrichtung gemäß einem Ausführungsbeispiel der Erfindung.

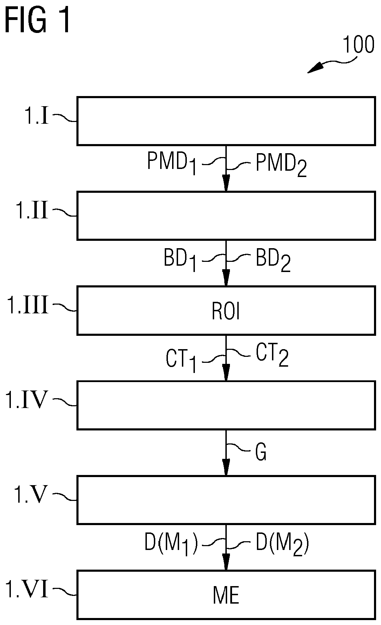

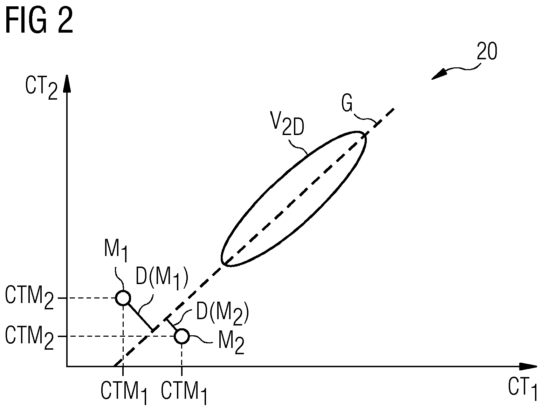

In FIG 1 ist ein Flussdiagramm 100 gezeigt, welches ein Verfahren zur Analyse eines Auswertungsbereichs ROI eines Untersuchungsbereichs eines Untersuchungsobjekts gemäß einem ersten Ausführungsbeispiel der Erfindung zeigt. Bei dem Schritt 1.I werden zwei Projektionsmessdatensätze PMD₁, PMD₂ von einem den Auswertungsbereich ROI umfassenden Untersuchungsbereich des Untersuchungsobjekts O erfasst. Die Projektionsmessdatensätze PMD₁, PMD₂ werden mit Hilfe einer Multi-Energie-CT-Aufnahme mit unterschiedlichen effektiven Röntgenenergiespektren von dem Untersuchungsbereich des Untersuchungsobjekts O erzeugt. Auf Basis der zwei Projektionsmessdatensätze PMD₁, PMD₂ werden bei dem Schritt 1.II zwei Bilddatensätze BD₁, BD₂ rekonstruiert, wobei jeweils ein Bilddatensatz einem der Projektionsmessdatensätze PMD₁, PMD₂ mit unterschiedlichem Röntgenspektrum zugeordnet ist. Bei dem Schritt 1.III werden Abschwächungswertepaare CT₁, CT₂ auf Basis der beiden Bilddatensätze BD₁, BD₂ in dem Auswertungsbereich ROI ermittelt, wobei die beiden Elemente der Abschwächungswertepaare CT₁, CT₂ jeweils einem gemeinsamen Bildpunkt der beiden Bilddatensätze BD₁, BD₂ zugeordnet sind. Die Abschwächungswertepaare CT₁, CT₂ der zwei Bilddatensätze BD₁, BD₂ bilden eine zweidimensionale Verteilung V_{2D}. Die zweidimensionale Verteilung V_{2D} der Abschwächungswerte CT₁, CT₂ ist in FIG 2 in einem Schaubild 20 veranschaulicht. Wie in FIG 2 zu erkennen ist, bilden die Abschwächungswerte CT₁, CT₂ eine näherungsweise elliptische Verteilung aus. Bei dem Schritt 1.IV wird dann auf Basis der zweidimensionalen Verteilung V_{2D} eine die Abschwächungswerte CT₁, CT₂ charakterisierende Gerade G (siehe FIG 2) ermittelt. Die Gerade G kann zum Beispiel mit Hilfe einer Regressionsformel oder einem Hauptkomponentenanalyseverfahren ermittelt werden.

Bei dem Schritt 1.V wird zur Ermittlung der Materialeigenschaften des Auswertungsbereichs ROI auf Basis der ermittelten Geraden G und einer die gesuchte Materialeigenschaft betreffenden Nebenbedingung eine Vergleichsrechnung durchgeführt. Hierbei werden als Nebenbedingung Abschwächungswertepaare CTM₁, CTM₂ unterschiedlicher Kandidaten-Materialien M₁, M₂ verwendet. Zur Ermittlung einer den Auswertungsbereich ROI betreffenden Materialeigenschaft werden Abstände D(M₁), D(M₂) zwischen den Abschwächungswertepaaren CTM₁, CTM₂ der Kandidaten-Materialien M₁, M₂ und der Geraden G berechnet (siehe auch FIG 2). Anschließend wird bei dem Schritt 1.VI bei der Ermittlung der Materialeigenschaft ME dasjenige Kandidaten-Material als Material des Auswertungsbereichs ROI ermittelt, dessen Abstand zu der Geraden G am kleinsten ist.

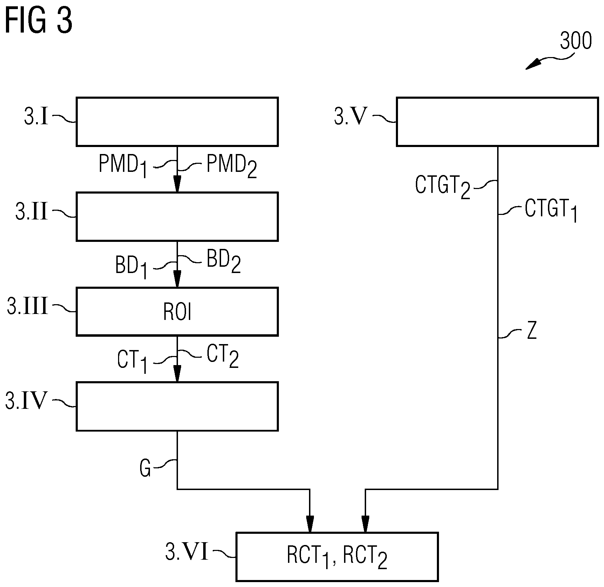

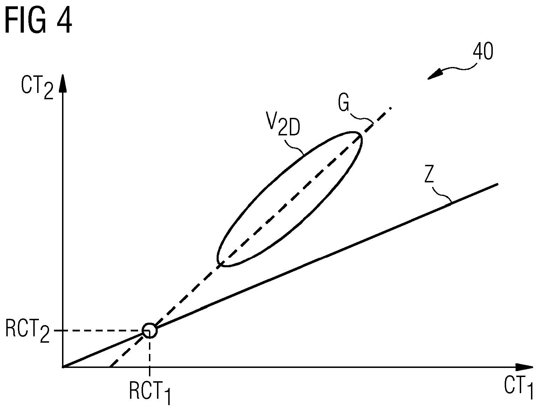

In FIG 3 ist ein Flussdiagramm 300 gezeigt, welches ein Verfahren zur Analyse eines Auswertungsbereichs ROI eines Untersuchungsbereichs eines Untersuchungsobjekts gemäß einem zweiten Ausführungsbeispiel der Erfindung zeigt. Das in FIG 3 veranschaulichte Verfahren wird wieder mit Hilfe eines Schaubilds 40, welches in FIG 4 dargestellt ist, illustriert. Bei dem Schritt 3.I werden zwei Projektionsmessdatensätze PMD₁, PMD₂ von dem Auswertungsbereich ROI des Untersuchungsbereichs des Untersuchungsobjekts erfasst. Die Projektionsmessdatensätze PMD₁, PMD₂ werden mit Hilfe einer Multi-Energie-CT-Aufnahme mit unterschiedlichen effektiven Röntgenenergiespektren von dem Untersuchungsbereich des Untersuchungsobjekts erzeugt. Auf Basis der zwei Projektionsmessdatensätze PMD₁, PMD₂ werden bei dem Schritt 3.II zwei Bilddatensätze BD₁, BD₂ rekonstruiert, wobei jeweils ein Bilddatensatz einem der Projektionsmessdatensätze mit unterschiedlichem Röntgenspektrum zugeordnet ist. Bei dem Schritt 3.III werden Abschwächungswertepaare CT₁, CT₂ auf Basis der beiden Bilddatensätze BD₁, BD₂ ermittelt, wobei die beiden Elemente der Abschwächungswertepaare CT₁, CT₂ jeweils einem gemeinsamen Bildpunkt der beiden Bilddatensätze BD₁, BD₂ in dem Auswertungsbereich ROI zugeordnet sind. Die Abschwächungswertepaare CT₁, CT₂ der zwei Bilddatensätze BD₁, BD₂ bilden eine zweidimensionale Verteilung V_{2D}. Die zweidimensionale Verteilung V_{2D} der Abschwächungswerte CT₁, CT₂ ist in FIG 4 in einem Schaubild 40 veranschaulicht. Wie in FIG 4 zu erkennen ist, bilden die Abschwächungswerte CT₁, CT₂ eine näherungsweise elliptische Verteilung aus. Bei dem Schritt 3.IV wird dann auf Basis der zweidimensionalen Verteilung V_{2D} eine die Abschwächungswerte CT₁, CT₂ charakterisierende Gerade G ermittelt. Die Gerade G kann zum Beispiel mit Hilfe einer Regressionsformel ermittelt werden.

Bei dem Schritt 3.V wird nun als Nebenbedingung eine Gerade Z verwendet, welche Abschwächungswertepaare CTGT₁, CTGT₂ unterschiedlicher Arten von Kandidaten-Gewebestrukturen repräsentiert. Diese Gewebestrukturen können zum Beispiel unterschiedliche Arten von Zysten umfassen, denen unterschiedliche Abschwächungswerte zugeordnet sind.

Bei dem Schritt 3.VI wird wiederum eine den Auswertungsbereich ROI des Untersuchungsbereichs betreffende Materialeigenschaft ermittelt. Dies wird erreicht durch das Ermitteln eines realen Abschwächungswertepaares RCT₁, RCT₂, dessen in dem Schaubild 40 in FIG 4 zugeordneter Punkt als Schnittpunkt der die Verteilung der Abschwächungswerte CT₁, CT₂ charakterisierenden Geraden G und der Geraden Z, welche Abschwächungswertepaare CTGT₁, CTGT₂ unterschiedlicher Arten von Kandidaten-Gewebestrukturen repräsentiert, ermittelt wird. Die Position des ermittelten Schnittpunkts auf der Geraden Z gibt Auskunft über den Typ der untersuchten Gewebestruktur.

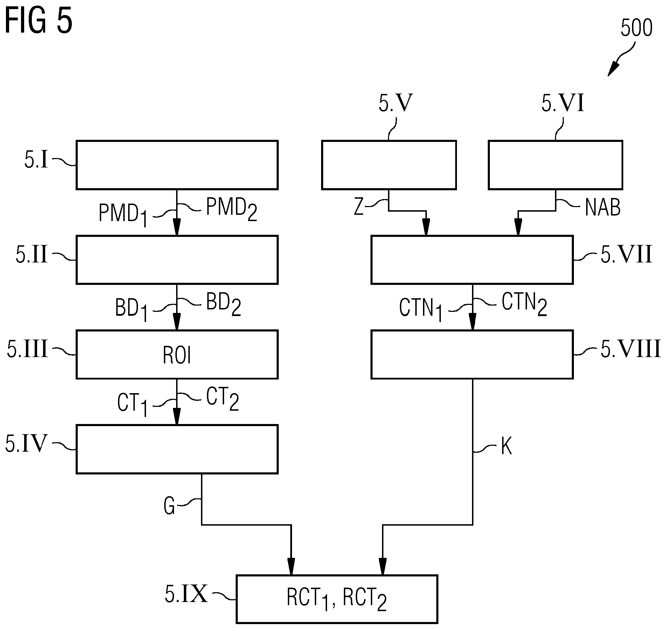

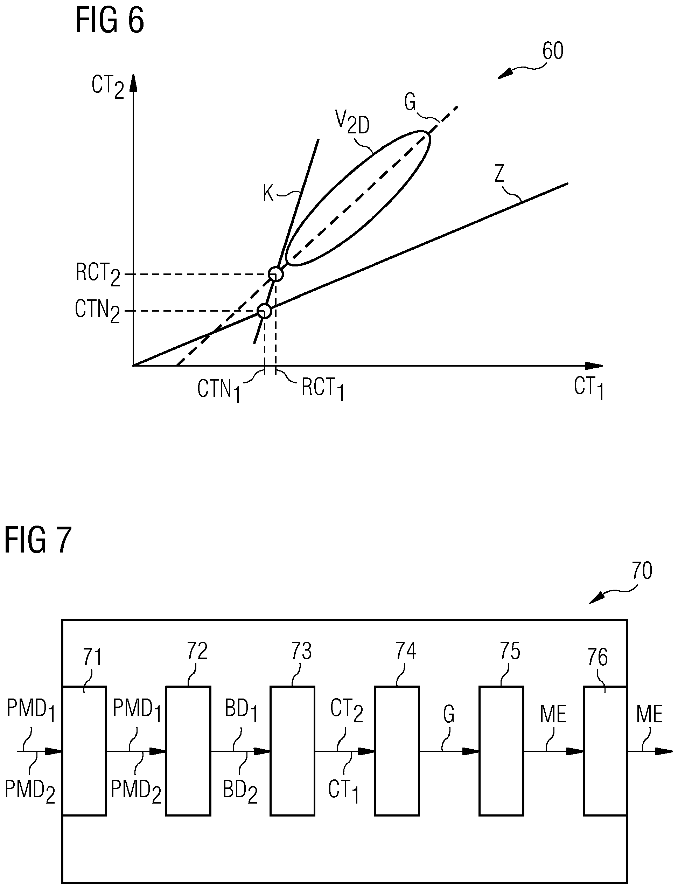

In FIG 5 ist ein Flussdiagramm 500 gezeigt, welches ein Verfahren zur Analyse eines Auswertungsbereichs ROI eines Untersuchungsbereichs eines Untersuchungsobjekts gemäß einem dritten Ausführungsbeispiel der Erfindung zeigt. Das in FIG 5 veranschaulichte Verfahren wird wieder mit Hilfe eines Schaubilds 60, welches in FIG 6 dargestellt ist, illustriert. Die Schritte 5.1 bis 5.IV entsprechen den Schritten 1.I bis 1.IV bzw. 3.I bis 3.IV und werden daher im Zusammenhang mit FIG 5 nicht nochmals ausführlich beschrieben. Bei dem Schritt 5.V wird ähnlich wie bei dem Schritt 3.V des zweiten Ausführungsbeispiels als Nebenbedingung eine Gerade Z verwendet, welche Abschwächungswertepaare CTGT₁, CTGT₂ unterschiedlicher Arten von Kandidaten-Gewebestrukturen repräsentiert. Diese Gewebestrukturen können zum Beispiel unterschiedliche Arten von Zysten umfassen, denen unterschiedliche Abschwächungswerte zugeordnet sind. Weiterhin wird zur Gewinnung von zusätzlichen Informationen bei dem Schritt 5.VI ein Nativbild NAB von dem Untersuchungsbereich aufgenommen.

Auf Basis dieses Nativbilds NAB und der Geraden Z wird bei dem Schritt 3.VII ein Wertpaar CTN₁, CTN₂ ermittelt, welches ein das Nativbild NAB repräsentierendes Abschwächungswertepaar darstellt. Bei dem Schritt 5.VIII wird dann auf Basis der Kenntnis des für die bei der spektralen Bildaufnahme bei dem Schritt 5.I verwendeten Kontrastmittels eine Gerade K ermittelt, welche durch den Punkt auf der Geraden Z verläuft, der durch das Wertepaar CTN₁, CTN₂ definiert ist, welches ein das Nativbild repräsentierendes Abschwächungswertepaar darstellt. Die Gerade K charakterisiert die Kontrastmittelaufnahme des Auswertungsbereichs ROI. Bei dem Schritt 5.IX wird schließlich als eine den Auswertungsbereich ROI betreffende Materialeigenschaft ein reales Abschwächungswertepaar RCT₁, RCT₂ als Schnittpunkt der die Kontrastmittelaufnahme des Auswertungsbereichs ROI charakterisierenden Geraden K und der die Verteilung der Abschwächungswerte CT₁, CT₂ charakterisierenden Geraden G ermittelt. Liegt ein Abstand zwischen dem ermittelten Punkt des realen Abschwächungswertepaares RCT₁, RCT₂ und dem Punkt, welcher durch das Wertepaar CTN₁, CTN₂ gebildet wird, welches das Nativbild NAB repräsentiert, vor, so kann daraus geschlossen werden, dass ein Tumor in dem Auswertungsbereich ROI vorhanden ist. Die Größe des Abstands zwischen dem ermittelten Punkt des realen Abschwächungswertepaares RCT₁, RCT₂ und dem Punkt, welcher durch das Wertepaar CTN₁, CTN₂ gebildet wird, welches das Nativbild NAB repräsentiert, kann als Information hinsichtlich des Typs des detektierten Tumors genutzt werden.

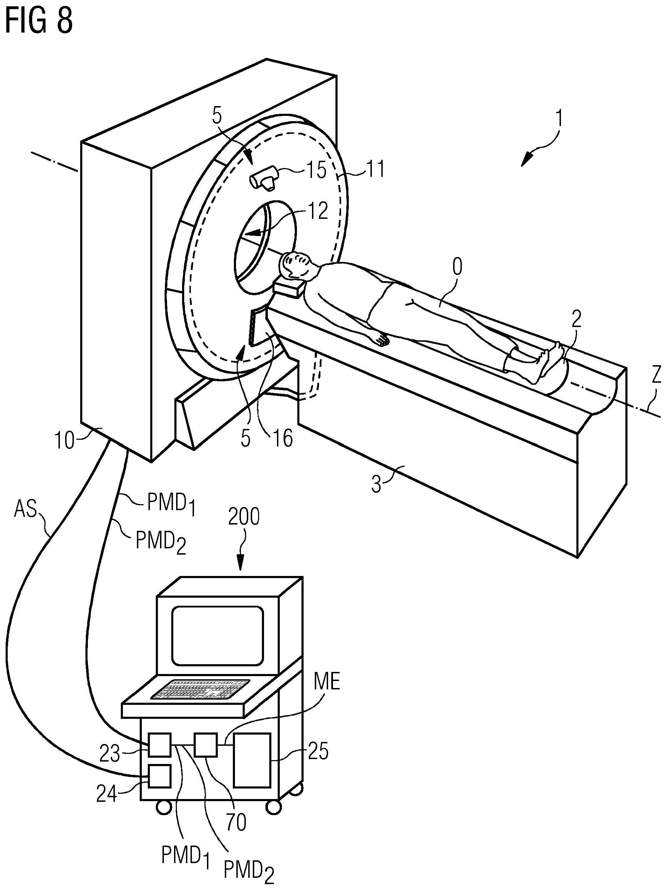

In FIG 7 ist ein Blockdiagramm gezeigt, welches schematisch eine Bildauswerteeinrichtung 70 gemäß einem Ausführungsbeispiel der Erfindung veranschaulicht. Die Bildauswerteeinrichtung 70 umfasst eine Projektionsmessdatenerfassungseinheit 71, welche dazu eingerichtet ist, zwei Projektionsmessdatensätze PMD₁, PMD₂, welche mit Hilfe einer Multi-Energie-CT-Aufnahme mit unterschiedlichen effektiven Röntgenenergiespektren von einem Untersuchungsbereich eines Untersuchungsobjekts O (siehe FIG 8) erzeugt wurden, zu erfassen. Teil der Bildauswerteeinrichtung 70 ist auch eine Bilddatenrekonstruktionseinheit 72, welche dazu dient, zwei Bilddatensätze BD₁, BD₂ auf Basis der zwei Projektionsmessdatensätze PMD₁, PMD₂ zu rekonstruieren.

Die Bilddatensätze BD₁, BD₂ werden an eine Paar-Ermittlungseinheit 73 übermittelt, welche Abschwächungswertepaaren CT₁, CT₂, die jeweils einem gemeinsamen Bildpunkt der beiden Bilddatensätze BD₁, BD₂ zugeordnet sind, ermittelt. Die Abschwächungswertepaare CT₁, CT₂ bilden eine zweidimensionale Verteilung der Abschwächungswertepaare CT₁, CT₂ der zwei Bilddatensätze BD₁, BD₂ in einem Auswertungsbereich ROI des Untersuchungsbereichs. Teil der Bildauswerteeinrichtung 70 ist auch eine Geraden-Ermittlungseinheit 74, welche dazu eingerichtet ist, eine die Verteilung der Abschwächungswerte CT₁, CT₂ charakterisierende Gerade G zu ermitteln. Auf Basis der Geraden G und entsprechender Nebenbedingungen wird dann in einer Materialeigenschaft-Ermittlungseinheit 75 eine den Untersuchungsbereich betreffende Materialeigenschaft ME ermittelt und über eine Ausgabeeinheit 76 ausgegeben.

In FIG 8 ist schematisch ein Computertomographiesystem (CT-System) 1 mit einer Bildauswerteeinrichtung 70 gemäß einem Ausführungsbeispiel der Erfindung dargestellt. Das CT-System 1 wird zur Aufnahme von Projektionsmessdaten PMD₁, PMD₂ von einem Untersuchungsbereich eines Patienten verwendet.

Das CT-System 1 besteht dabei im Wesentlichen aus einer Scaneinheit 10, in welcher an einer Gantry 11 eine Projektionsdatenakquisitionseinheit 5 mit einem Detektor 16 und einer dem Detektor 16 gegenüberliegenden Röntgenquelle 15 um einen Messraum 12 umläuft. Vor der Scaneinheit 10 befindet sich eine Patientenlagerungseinrichtung 3 bzw. ein Patiententisch 3, dessen oberer Teil 2 mit einem darauf befindlichen Patienten O zu der Scaneinheit 10 verschoben werden kann, um den Patienten O durch den Messraum 12 hindurch relativ zum Detektorsystem 16 zu bewegen. Angesteuert werden die Scaneinheit 10 und der Patiententisch 3 durch eine Steuerungseinrichtung 200, von der aus über eine übliche Steuerschnittstelle 24 Akquisitionssteuersignale AS kommen, um das gesamte System gemäß vorgegebener Messprotokolle in der herkömmlichen Weise anzusteuern. Durch die Bewegung des Patienten O entlang der z-Richtung, welche der Systemachse z längs durch den Messraum 12 entspricht, und den gleichzeitigen Umlauf der Röntgenquelle 15 ergibt sich für die Röntgenquelle 15 relativ zum Patienten O während der Messung eine Helixbahn. Parallel läuft dabei immer gegenüber der Röntgenquelle 15 der Detektor 16 mit, um Projektionsmessdaten PMD₁, PMD₂ zu erfassen, die dann zur Rekonstruktion von Volumen- und/oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen multi-energetischen Projektionsmessdaten PMD₁, PMD₂ erfasst werden, um ein Schnittbild an dieser z-Position zu rekonstruieren oder um aus den Projektionsdaten mehrerer z-Positionen Volumenbilddaten zu rekonstruieren. Das erfindungsgemäße Verfahren ist grundsätzlich auch an anderen CT-Systemen, z. B. einem Dual-Source-Dual-Energy-CT oder mit einem einen vollständigen Ring bildenden Detektor, einsetzbar.

Der Detektor 16 ist in dem in FIG 8 gezeigten Ausführungsbeispiel ein quantenzählender Detektor, mit dem erfasste Röntgenquanten nach der Röntgenenergie aufgelöst erfasst werden können. Mit Hilfe des quantenzählenden Detektors lassen sich die erfassten Röntgenquanten unterschiedlichen Röntgenenergiespektren zuordnen, so dass zum Beispiel zwei Dual-Energie-CT-Projektionsmessdatensätze PMD₁, PMD₂ erzeugt werden.

Die vom Detektor 16 akquirierten Mess-Projektionsdatensätze PMD₁, PMD₂ von einem Untersuchungsbereich des Patienten O werden über eine Rohdatenschnittstelle 23 an die Steuerungseinrichtung 200 übergeben. Diese Projektionsmessdatensätze PMD₁, PMD₂ werden dann von einer Auswerteeinrichtung 70 gemäß einem Ausführungsbeispiel der Erfindung auf die in FIG 1 bis FIG 6 beschriebene Art und Weise verarbeitet, wobei Materialeigenschaften ME eines Auswertungsbereichs ROI ermittelt werden und in einer Speichereinrichtung 25 abgespeichert werden bzw. auf einem Bildschirm des CT-Systems 1 angezeigt werden.

Die Projektionsmessdatensätze PMD₁, PMD₂ werden von der Bildauswerteeinrichtung 70 weiterverarbeitet und analysiert. Die dabei ermittelten Materialeigenschaften ME werden in einer Speichereinheit 25 abgespeichert und auf einer Anzeigeeinheit der Steuerungseinrichtung 200 angezeigt.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zur Analyse eines Auswertungsbereichs (ROI) eines Untersuchungsbereichs eines Untersuchungsobjekts (O), aufweisend die Schritte:
- Erfassen von mindestens zwei Projektionsmessdatensätzen (PMD₁, PMD₂), welche mit Hilfe einer Multi-Energie-CT-Aufnahme mit unterschiedlichen effektiven Röntgenenergiespektren von dem Untersuchungsbereich des Untersuchungsobjekts (O) erzeugt wurden,
- Rekonstruieren von zwei Bilddatensätzen (BD₁, BD₂) auf Basis der mindestens zwei Projektionsmessdatensätze (PMD₁, PMD₂),
- Ermitteln von Abschwächungswertepaaren (CT₁, CT₂), welche jeweils einem gemeinsamen Bildpunkt der beiden Bilddatensätze (BD₁, BD₂) in dem Auswertungsbereich (ROI) zugeordnet sind,
- Ermitteln einer eine zweidimensionale Verteilung (V_{2D}) der Abschwächungswerte (CT₁, CT₂) charakterisierenden Geraden (G),
- Ermitteln einer den Auswertungsbereich (ROI) betreffenden Materialeigenschaft (ME) auf Basis der ermittelten Geraden (G) und einer die gesuchte Materialeigenschaft (ME) betreffenden Nebenbedingung.

2. Verfahren nach Anspruch 1, wobei zunächst mehr als zwei Projektionsmessdatensätze (PMD₁, PMD₂)erfasst werden und die mehr als zwei Projektionsmessdatensätze (PMD₁, PMD₂)auf genau zwei Projektionsmessdatensätze (PMD₁, PMD₂)reduziert werden.

3. Verfahren nach Anspruch 1 oder 2, wobei der Auswertungsbereich (ROI) eine Läsion, vorzugsweise eine hypodense Läsion, und zusätzlich die Läsion umgebendes Gewebe umfasst.

4. Verfahren nach einem der vorstehenden Ansprüche, wobei
- als Nebenbedingung Abschwächungswertepaare (CTM₁, CTM₂) unterschiedlicher Kandidaten-Materialien (M₁, M₂) verwendet werden und
- das Ermitteln einer den Auswertungsbereich (ROI) betreffenden Materialeigenschaft (ME) das Ermitteln eines der Kandidaten-Materialien (M₁, M₂) als Bestandteil des Auswertungsbereichs (ROI) umfasst, dessen Abschwächungswertepaar (CTM₁, CTM₂) der Geraden (G) am nächsten kommt.

5. Verfahren nach einem der Ansprüche 1 bis 3, wobei
- als Nebenbedingung eine Gerade (Z) verwendet wird, welche Abschwächungswertepaare (CTGT₁, CTGT₂) unterschiedlicher Arten von Kandidaten-Gewebestrukturen repräsentiert und
- das Ermitteln einer den Auswertungsbereich (ROI) betreffenden Materialeigenschaft (ME) das Ermitteln eines realen Abschwächungswertepaares (RCT₁, RCT₂) umfasst, welches als Schnittpunkt der die Verteilung der Abschwächungswerte (CT₁, CT₂) charakterisierenden Geraden (G) und der Geraden (Z), welche Abschwächungswertepaare (CTGT₁, CTGT₂) unterschiedlicher Arten von Kandidaten-Gewebestrukturen repräsentiert, ermittelt wird.

6. Verfahren nach einem der Ansprüche 1 bis 3, wobei
- als Nebenbedingung eine Gerade (Z) verwendet wird, welche Abschwächungswertepaare (CTGT1, CTGT2) unterschiedlicher Arten von Kandidaten-Gewebestrukturen repräsentiert und
- zusätzlich Nativbilddaten (NAB) von dem Untersuchungsbereich erzeugt werden,
- auf Basis des Nativbildes (NAB) ein Abschwächungswertepaar (CTN1, CTN2) auf der Geraden (Z), welche Abschwächungswertepaare (CTGT1, CTGT2) unterschiedlicher Arten von Kandidaten-Gewebestrukturen repräsentiert, ermittelt wird, wobei das ermittelte Abschwächungswertepaar (CTN1, CTN2) den Auswertungsbereich (ROI) in dem Nativbild (NAB) charakterisiert,
- eine die Kontrastmittelaufnahme des Auswertungsbereichs (ROI) charakterisierende Gerade (K) ermittelt wird, welche durch den Punkt des den Auswertungsbereich (ROI) in dem Nativbild (NAB) charakterisierenden Abschwächungswertepaars (CTN₁, CTN₂) verläuft, und
- das Ermitteln einer den Auswertungsbereich (ROI) betreffenden Materialeigenschaft (ME) das Ermitteln eines realen Abschwächungswertepaares (RCT₁, RCT₂) umfasst, welches den Schnittpunkt der die Kontrastmittelaufnahme des Auswertungsbereichs (ROI) charakterisierenden Geraden (K) und der die Verteilung der Abschwächungswerte (CT₁, CT₂) charakterisierenden Geraden (G) repräsentiert.

7. Verfahren nach einem der vorstehenden Ansprüche, wobei auf Basis der ermittelten Materialeigenschaft (ME) ermittelt wird, ob in dem Auswertungsbereich (ROI) ein Tumor oder eine Zyste vorliegt.

8. Verfahren nach Anspruch 5 und 7, wobei in Abhängigkeit davon, ob ein Wert des realen Abschwächungswertepaares (RCT₁, RCT₂) einen vorbestimmten Schwellwert überschreitet, ermittelt wird, ob in dem Auswertungsbereich (ROI) ein Tumor vorliegt.

9. Verfahren nach Anspruch 8, wobei die verwendeten Abschwächungswertepaare (CT₁, CT₂) auf Basis von monoenergetischen Bilddaten oder Basismaterialbilddaten erzeugt werden.

10. Verfahren nach Anspruch 6 und 7, wobei in Abhängigkeit davon, ob der Punkt des realen Abschwächungswertepaares (RCT₁, RCT₂) und der Punkt des den Auswertungsbereich (ROI) in dem Nativbild (NAB) charakterisierenden Abschwächungswertepaars (CTN₁, CTN₂) voneinander entfernt liegen, ermittelt wird, ob ein Tumor oder eine Zyste in dem Auswertungsbereich (ROI) vorliegen.

11. Verfahren nach Anspruch 10, wobei in Abhängigkeit von dem Abstand zwischen dem Punkt des realen Abschwächungswertepaares (RCT₁, RCT₂) und dem Punkt des den Auswertungsbereich (ROI) in dem Nativbild (NAB) charakterisierenden Abschwächungswertepaars (CTN₁, CTN₂) die Art eines vorliegenden Tumortyps ermittelt wird.

12. Verfahren nach einem der vorstehenden Ansprüche, wobei der Auswertungsbereich (ROI) hinsichtlich einer der folgenden Strukturen untersucht wird:
- eine gewöhnliche Zyste,
- eine eingeblutete Zyste,
- Metastasen,
- einen Primärtumor.

13. Bildauswerteeinrichtung (70), aufweisend:
- eine Projektionsmessdatenerfassungseinheit (71) zum Erfassen von mindestens zwei Projektionsmessdatensätzen (PMD1, PMD2), welche mit Hilfe einer Multi-Energie-CT-Aufnahme mit unterschiedlichen effektiven Röntgenenergiespektren von einem Untersuchungsbereich eines Untersuchungsobjekts (O) erzeugt wurden,
- eine Bilddatenrekonstruktionseinheit (72) zum Rekonstruieren von zwei Bilddatensätzen (BD1, BD2) auf Basis der mindestens zwei Projektionsmessdatensätze (PMD1, PMD2),
- eine Paar-Ermittlungseinheit (73) zum Ermitteln von Abschwächungswertepaaren (CT1, CT2), welche jeweils einem gemeinsamen Bildpunkt der beiden Bilddatensätze (BD1, BD2) in einem Auswertungsbereich (ROI) des Untersuchungsbereichs zugeordnet sind,
- einer Geraden-Ermittlungseinheit (74) zum Ermitteln einer eine zweidimensionale Verteilung (V2D) der Abschwächungswerte (CT1, CT2) charakterisierenden Geraden (G),
- eine Materialeigenschaft-Ermittlungseinheit (75) zum Ermitteln einer den Auswertungsbereich (ROI) betreffenden Materialeigenschaft (ME) auf Basis der ermittelten Geraden (G) und einer die gesuchte Materialeigenschaft (ME) betreffenden Nebenbedingung.

14. Computertomographiesystem (1), aufweisend:
- eine Multi-Energie-Scaneinheit (10) zum dual-energetischen Abtasten eines Untersuchungsbereichs eines zu untersuchenden Objekts (O),
- eine Steuerungseinrichtung (200) zum Ansteuern der Scaneinheit (10),
- eine Bildauswerteeinrichtung (70) nach Anspruch 13.

15. Computerprogrammprodukt mit einem Computerprogramm, welches direkt in eine Speichereinheit (25) eines Computertomographiesystems (1) ladbar ist, mit Programmabschnitten, um alle Schritte eines Verfahrens nach einem der Ansprüche 1 bis 12 auszuführen, wenn das Computerprogramm in dem Computertomographiesystem (1) ausgeführt wird.

16. Computerlesbares Medium, auf welchem von einer Prozesseinheit einlesbare und ausführbare Programmabschnitte gespeichert sind, um alle Schritte eines Verfahrens nach einem der Ansprüche 1 bis 12 auszuführen, wenn die Programmabschnitte von der Prozesseinheit ausgeführt werden.

## Claims

1. Method for analysing an evaluation region (ROI) of an examination region of an examination object (O), having the steps:
- detecting at least two projection measurement data records (PMD₁, PMD₂), which were generated from the examination region of the examination object (O) with the aid of a multi energy CT recording using different effective x-ray energy spectra,
- reconstructing two image data records (BD₁, BD₂) based on the at least two projection measurement data records (PMD₁, PMD₂),
- determining attenuation value pairs (CT₁, CT₂), which are each assigned to a shared pixel of the two image data records (BD₁, BD₂) in the evaluation region (ROI),
- determining a straight line (G) which characterises a two-dimensional distribution (V_{2D}) of the attenuation values (CT₁, CT₂) ,
- determining a material property (ME) relating to the evaluation region (ROI) on the basis of the determined straight line (G) and an auxiliary condition relating to the sought material property (ME).

2. Method according to claim 1, wherein initially more than two projection measurement data records (PMD₁, PMD₂)are detected and the more than two projection measurement data records (PMD₁, PMD₂)are reduced to precisely two projection measurement data records (PMD₁, PMD₂).

3. Method according to claim 1 or 2, wherein the evaluation region (ROI) comprises a lesion, preferably a hypodense lesion, and in addition tissue surrounding the lesion.

4. Method according to one of the preceding claims, wherein
- attenuation value pairs (CTM₁, CTM₂) of different candidate materials (M₁, M₂) are used as an auxiliary condition and
- the determination of a material property (ME) relating to the evaluation region (ROI) comprises the determination of one of the candidate materials (M₁, M₂) as an integral part of the evaluation region (ROI), the attenuation value pair (CTM₁, CTM₂) of which is closest to the straight line (G).

5. Method according to one of claims 1 to 3, wherein
- a straight line (Z) is used as an auxiliary condition, said straight line representing attenuation value pairs (CTGT₁, CTGT₂) of different types of candidate tissue structures and
- the determination of a material property (ME) relating to the evaluation region (ROI) comprises the determination of a real attenuation value pair (RCT₁, RCT₂), which is determined as a point of intersection of the straight line (G) which characterises the distribution of the attenuation values (CT₁, CT₂), and straight line (Z) which represents attenuation value pairs (CTGT₁, CTGT₂) of different types of candidate tissue structures.

6. Method according to one of claims 1 to 3, wherein
- a straight line (Z) is used as an auxiliary condition, which represents attenuation value pairs (CTGT₁, CTGT₂) of different types of candidate tissue structures and
- in addition native image data (NAB) of the examination region is generated,
- on the basis of the native image (NAB) an attenuation value pair (CTN₁, CTN₂) is determined on the straight line (Z), which represents attenuation value pairs (CTGT₁, CTGT₂) of different types of candidate tissue structures, wherein the determined attenuation value pair (CTN₁, CTN₂) characterises the evaluation region (ROI) in the native image (NAB),
- a straight line (K) characterising the contrast agent recording of the evaluation region (ROI) is determined, which runs through the point of the attenuation value pair (CTN₁, CTN₂) characterising the evaluation region (ROI) in the native image (NAB), and
- the determination of a material property (ME) relating to the evaluation region (ROI) comprises the determination of a real attenuation value pair (RCT₁, RCT₂), which represents the point of intersection of the straight line (K) characterising the contrast agent recording of the evaluation region (ROI) and which represents the distribution of the straight line (G) characterising the distribution of the attenuation values (CT₁, CT₂) .

7. Method according to one of the preceding claims, wherein it is determined on the basis of the determined material property (ME) whether a tumour or a cyst is present in the evaluation region (ROI).

8. Method according to claim 5 and 7, wherein as a function of whether a value of the real attenuation value pair (RCT₁, RCT₂) exceeds a predetermined threshold value, it is determined whether a tumour is present in the evaluation region (ROI).

9. Method according to claim 8, wherein the used attenuation value pairs (CT₁, CT₂) are generated on the basis of monoenergetic image data or base material image data.

10. Method according to claim 6 and 7, wherein as a function of whether the point of the real attenuation value pair (RCT₁, RCT₂) and the point of the attenuation value pair (CTN₁, CTN₂) characterising the evaluation region (ROI) in the native image (NAB) are at a distance from one another, it is determined whether a tumour or a cyst is present in the evaluation region (ROI) .

11. Method according to claim 10, wherein the type of existing tumour type is determined as a function of the distance between the point of the real attenuation value pair (RCT₁, RCT₂) and the point of the attenuation value pair (CTN₁, CTN₂) characterising the evaluation region (ROI) in the native image (NAB).

12. Method according to one of the preceding claims, wherein the evaluation region (ROI) is examined in respect of one of the following structures:
- a typical cyst
- a cyst supplied with blood,
- metastases,
- a primary tumour.

13. Image evaluation device (70), having:
- a projection measurement data detection unit (71) for detecting at least two projection measurement data records (PMD₁, PMD₂), which were generated from an examination region of an examination object (O) with the aid of a multi energy CT recording using different effective x-ray energy spectra,
- an image reconstruction unit (72) for reconstructing two image data records (BD₁, BD₂) based on the at least two projection measurement data records (PMD₁, PMD₂),
- a pair determination unit (73) for determining attenuation value pairs (CT₁, CT₂), which are each assigned to a shared pixel of the two image data records (BD₁, BD₂) in an evaluation region (ROI) of the examination region,
- a straight line determination unit (74) for determining a straight line (G) which characterises a two-dimensional distribution (V_{2D}) of the attenuation values (CT₁, CT₂),
- a material property determination unit (75) for determining a material property (ME) relating to the evaluation region (ROI) on the basis of the determined straight line (G) and an auxiliary condition relating to the sought material property (ME) .

14. Computed tomography system (1), having:
- a multi energy scan unit (10) for the dual energy scanning of an examination region of an object (O) to be examined,
- a control device (200) for controlling the scan unit (10),
- an image evaluation device (70) according to claim 13.

15. Computer program product with a computer program, which can be loaded directly into a storage unit (25) of a computed tomography system (1), with program segments in order to execute all steps of a method according to one of claims 1 to 12, if the computer program is executed in the computed tomography system (1).

16. Computer readable medium, upon which program segments which can be read in and executed by a process unit are stored, in order to execute all steps of a method according to one of claims 1 to 12, if the program segments are executed by the process unit.

## Revendications

1. Procédé d'analyse d'une partie (ROI) à évaluer d'une partie à examiner d'un objet (O) à examiner, comprenant les stades :
- saisie d'au moins deux ensembles (PMD₁, PMD₂)de donnée de mesure de projection, qui ont été produits à l'aide d'un enregistrement CT multi-énergie avec des spectres d'énergie de rayons-X effectifs différents de la partie à examiner de l'objet (O) à examiner,
- reconstruction de deux ensembles (BD₁, BD₂) de données d'image sur la base des au moins deux ensembles (PMD₁, PMD₂)de données de mesure de projection,
- détermination de paires (CT₁, CT₂) de valeurs d'affaiblissement, qui sont associées dans la partie (ROI) d'évaluation respectivement à un point image commun des deux ensembles (BD₁, BD₂) de données d'image,
- détermination d'une droite (G) caractérisant une répartition (V_{2D}) en deux dimensions des valeurs (CT₁, CT₂) d'affaiblissement,
- détermination d'une propriété (ME) de matière concernant la partie (ROI) à évaluer sur la base de la droite (G) déterminée et d'une condition secondaire concernant la propriété (ME) de matière recherchée.

2. Procédé suivant la revendication 1, dans lequel on saisit d'abord plus de deux ensembles (PMD₁, PMD₂)de données de mesure de projection et on réduit les plus de deux ensembles (PMD₁, PMD₂)de données de mesure de projection à exactement deux ensembles (PMD₁, PMD₂)de données de mesure de projection.

3. Procédé suivant la revendication 1 ou 2, dans lequel la partie (ROI) à évaluer comprend une lésion, de préférence une lésion hypodense, et en outre du tissu entourant la lésion.

4. Procédé suivant l'une des revendications précédentes, dans lequel
- on utilise comme condition secondaire des paires (CTM₁, CTM₂) de valeurs d'affaiblissement de matières (M₁, M₂) candidates différentes et
- la détermination d'une propriété (ME) de matière concernant la partie (ROI) à évaluer comprend la détermination comme constituant de la partie (ROI) à évaluer de l'une des matières (M₁, M₂) candidates, dont la paire (CTM₁, CTM₂) de valeurs d'affaiblissement se rapproche le plus de la droite (G).

5. Procédé suivant l'une des revendications 1 à 3, dans lequel
- on utilise comme condition secondaire une droite (Z), qui représente des paires (CTGT₁, CTGT₂) de valeurs d'affaiblissement de types différents de structures de tissu candidates et
- la détermination d'une propriété (ME) de matière concernant la partie (ROI) à évaluer comprend la détermination d'une paire (RCT₁, RCT₂) de valeurs d'affaiblissement réelle, que l'on détermine comme point d'intersection de la droite (G) caractérisant la répartition des valeurs (CT₁, CT₂) d'affaiblissement et de la droite (Z), qui représente des paires (CTGT₁, CTGT₂) de valeurs d'affaiblissement de types différents de structures de tissu candidates.

6. Procédé suivant l'une des revendications 1 à 3, dans lequel
- on utilise comme condition secondaire une droite (Z), qui représente des paires (CTGT1, CTGT2) de valeur d'affaiblissement de types différents de structures de tissu candidates et
- on produit en outre des données (NAB) de l'image native de la partie à examiner,
- on détermine sur la base de l'image (NAB) native une paire (CTN1, CTN2) de valeurs d'affaiblissement sur la droite (Z), qui représente des paires (CTGT1, CTGT2) de valeurs d'affaiblissement de types différents de structures de tissu candidates, la paire (CTN1, CTN2) de valeurs d'affaiblissement déterminée caractérisant la partie (ROI) à évaluer dans l'image (NAB) native,
- on détermine une droite (K) caractérisant l'absorption d'un agent de contraste de la partie (ROI) à évaluer, qui passe par le point de la paire (CTN₁, CTN₂) de valeur d'affaiblissement caractérisant la partie (ROI) à évaluer dans l'image (NAB) native, et
- la détermination d'une propriété (ME) de matière concernant la partie (ROI) à évaluer comprend la détermination d'une paire (RCT₁, RCT₂) de valeurs d'affaiblissement réelle, qui représente le point d'intersection de la droite (K) caractérisant l'absorption d'un agent de contraste de la partie (ROI) à évaluer et de la droite (G) caractérisant la répartition des valeurs (CT₁, CT₂) d'affaiblissement.

7. Procédé suivant l'une des revendications précédentes, dans lequel on détermine sur la base de la propriété (ME) de matière déterminée s'il y a une tumeur ou un kyste dans la partie (ROI) à évaluer.

8. Procédé suivant la revendication 5 et 7, dans lequel, en fonction du dépassement par une valeur de la paire (RCT₁, RCT₂) de valeurs d'affaiblissement réelle d'une valeur de seuil définie à l'avance, on détermine s'il y a une tumeur dans la partie (ROI) à évaluer.

9. Procédé suivant la revendication 8, dans lequel on produit les paires (CT₁, CT₂) de valeurs d'affaiblissement utilisées sur la base de données d'image mono-énergétiques ou de données d'image de matière de base.

10. Procédé suivant la revendication 6 et 7, dans lequel en fonction de l'éloignement l'un de l'autre du point de la paire (RCT₁, RCT₂) de valeurs d'affaiblissement réelle et du point de la paire (CTN₁, CTN₂) de valeurs d'affaiblissement caractérisant la partie (ROI) à évaluer dans l'image (NAB) native, on détermine s'il y a une tumeur ou un kyste dans la partie (ROI) à évaluer.

11. Procédé suivant la revendication 10, dans lequel en fonction de la distance entre le point de la paire (RCT₁, RCT₂) de valeurs d'affaiblissement réelle et le point de la paire (CTN₁, CTN₂) de valeurs d'affaiblissement caractérisant la partie (ROI) à évaluer dans l'image (NAB) native, on détermine la nature d'un type de tumeur présent.

12. Procédé suivant l'une des revendications précédentes, dans lequel on examine la partie (ROI) à évaluer en ce qui concerne l'une des structures suivantes :
- un kyste habituel,
- un kyste hématosé,
- des métastases,
- une tumeur primaire.

13. Dispositif (70) d'évaluation d'image, comportant :
- une unité (71) de saisie de données de mesure de projection pour la saisie d'au moins deux ensembles (PMD1, PMD2) de données de mesure de projection, qui ont été produits à l'aide d'un enregistrement CT multi-énergie avec des spectres d'énergie de rayons-X effectifs différents d'une partie à examiner d'un objet (O) à examiner,
- une unité (72) de reconstruction de données d'image pour la reconstruction de deux ensembles (BD1, BD2) de données d'image sur la base des au moins deux ensembles (PMD1, PMD2) de données de mesure de projection,
- une unité (73) de détermination de paire pour la détermination de paires (CT1, CT2) de valeur d'affaiblissement, qui sont associées chacune à un point d'image commun des deux ensembles (BD1, BD2) de données d'image dans une partie (ROI) à évaluer de la partie à examiner,
- une unité (74) de détermination de droite pour la détermination d'une droite (G) caractérisant une répartition (V2D) en deux dimensions des valeurs (CT1, CT2) d'affaiblissement,
- une unité (75) de détermination de propriété de matière pour la détermination d'une propriété (ME) de matière concernant la partie (ROI) à évaluer sur la base de la droite (G) déterminée et d'une condition secondaire concernant la propriété (ME) de matière recherchée.

14. Système (1) de tomographie par ordinateur, comportant :
- une unité (10) de scan multi-énergie pour le balayage énergétique dual d'une partie à examiner d'un objet (O) à examiner,
- un dispositif (200) de commande pour la commande de l'unité (10) de scan,
- un dispositif (70) d'évaluation d'image suivant la revendication 13.

15. Produit de programme d'ordinateur comprenant un programme d'ordinateur, qui peut se charger directement dans une unité (25) de mémoire d'un système (1) de tomodensitométrie par ordinateur, comprenant des parties de programme, pour effectuer tous les stades d'un procédé suivant l'une des revendications 1 à 12, lorsque le programme d'ordinateur est réalisé dans le système (1) de tomodensitométrie par ordinateur.

16. Support, déchiffrable par ordinateur, sur lequel des parties de programme, pouvant être lues et réalisées par une unité de process, sont mises en mémoire pour réaliser tous les stades d'un procédé suivant l'une des revendications 1 à 12, lorsque les parties de programme sont réalisées par l'unité de process.
